(19) Europäisches Patentamt

European Patent Office

Office européen des brevets

(11) Publication number: **0 318 072 B1**

# EUROPEAN PATENT SPECIFICATION

(45) Date of publication of patent specification: **14.04.93**

(51) Int. Cl.5: **B65D 90/52**, B65D 90/46, A62C 2/06, A62C 4/00

(21) Application number: **88202357.5**

(22) Date of filing: **21.10.88**

(54) **An antislosh, antistatic, and flame arresting structure for use with containers for holding flammable fluids.**

(30) Priority: **18.11.87 US 122141**

(43) Date of publication of application:
**31.05.89 Bulletin 89/22**

(45) Publication of the grant of the patent:
**14.04.93 Bulletin 93/15**

(84) Designated Contracting States:
**AT BE CH DE ES FR GB GR IT LI LU NL SE**

(56) References cited:
EP-A- 0 199 567
WO-A-88/02695
WO-A-89/03764

(73) Proprietor: **THE DOW CHEMICAL COMPANY**
**2030 Dow Center Abbott Road P.O. Box 1967**
**Midland Michigan 48640-1967(US)**

(72) Inventor: **McCullough, Francis P., Jr.**
**104 Fir Drive**
**Lake Jackson Texas 77566(US)**
Inventor: **Lane, Eckel R.**
**5608 Grouse Court**
**Midland Michigan 48640(US)**
Inventor: **Snelgrove, R. Vernon**
**Route 1 Box 112**
**Damon Texas 77430(US)**
Inventor: **Hall, David M.**
**855, Terrace Acres Drive**
**Aubrun Alabama 36830(US)**

(74) Representative: **Smulders, Theodorus A.H.J., Ir. et al**
**Vereenigde Octrooibureaux Nieuwe Parklaan 97**
**NL-2587 BN 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 318 072 B1

# EP 0 318 072 B1

## Description

The present invention relates to an antislosh, antistatic, flame arresting structure for use with containers holding flammable fluids and to a process for arresting flames in or about the container.

More particularly, the invention is concerned with resilient, shape reforming, lightweight, nonflammable structures of carbonaceous fibers having the ability to control or stop sloshing of fuel in a fuel container, act as a flame arrester, and prevent and/or dissipate the buildup of static electricity. The structures, are further characterized by having good chemical and hydrolytic inertness and are stable to long periods of exposure to the flammable fluids.

For many years the handling and use of flammable fluids such as liquid fuels, combustible gases and other combustible chemicals or intermediates have posed serious potential hazards of fire and explosion due to an electrostatic buildup during filling or unloading of containers, such as tanks, due to triboelectric generated discharge or due to sloshing of the liquids back and forth in the tanks during transportation of the tanks. Transporting tanks which partially contain flammable liquids, or which contain a small amount of liquid to the extent that the tank is essentially empty, poses the additional hazard of explosion of the liquid and/or gaseous by-products due to sparking or static electric discharge caused by the sloshing (rapid movement) of the liquid inside the tank. Methods used to prevent sparking and/or static discharge in the tank include padding the tank with an inert gas or adding a flame arresting system to the tank. In addition, physical grounding has been the customary technique for reducing this hazard. However, this technique has not eliminated the problem completely. In addition, human error and poor electrical connections have led to explosions resulting in loss of human life and equipment.

In more recent times, containers have been filled with a reticulated open cell polymeric foam in an effort to eliminate the above problems and hazards. One class of foam used heretofore is a polyether urethane which has good chemical resistance, but which has poor electrostatic dissipating properties and which is flammable in the presence of air. Electrostatic ignition could take place due to triboelectric generation if a container with the polyether urethane foam was to rupture due to stress or on impact with another object. The other class of foam used heretofore is a polyether urethane which has fairly good electrostatic dissipating properties, but has poor hydrolytic properties, poor solvent stability and is also fairly flammable in air. Both of the above classes of foams lose their structural properties when exposed to hydrocarbon fuels over a period of time and thus must be replaced regularly.

However, mere sloshing of a liquid is not the only source of electrostatic buildup, and therefore the control of sloshing and, in turn, the control of the electrostatic buildup created by the sloshing alone does not solve the other problems encountered in fuel tank usage. For example, since foam materials are not good electrical conductors, they do not discharge an electrostatic buildup such as that generated during filling and emptying of a container. In addition, such foams are weak structurally and exhibit slow degradation in the presence of most flammable liquids, especially hydrocarbon liquids. This instability problem is increased when a small amount of moisture is present in the container and contacts the foam. Another problem frequently encountered in the use of containers with flammable liquids and/or gases is cracking or rupturing of the containers due to impact or material fatigue. A crack or rupture in a container will allow the flammable fluids to freely escape and pose a dangerous and hazardous condition.

While some success has been obtained by the use of the above-mentioned foams, it would be advantageous for the industry to have a material which, in addition to preventing sloshing, is stable in the presence of flammable fluids, exhibits high electrical conductivity sufficient to dissipate electrostatic buildup and acts as a flame arrester in instances where a spark could occur. Further, it is desirable to have a material which is substantially flame resistant and has the capability, when used as an outer liner for a container, of greatly retarding the escape of a flammable liquid from the container should the container leak or rupture. Such a novel material is described hereinafter in the accompanying specification and examples. All percentages set forth hereinafter are in percent by weight unless otherwise specified.

In accordance with the present invention there is provided a lightweight, nonflammable structure comprising a multiplicity of irreversibly heat set, continuous or staple, carbonaceous fibers, in the form of a wool-like fluff, batting, webbing or the like.

The fibers are formed by a partial carbonization of stabilized polymeric fibers or fabric or some other stabilized carbon fiber precursor material, such as acrylic fibers or pitch fibers, under conditions to impart a substantially irreversible, nonlinear, e.g., sinusoidal or coil-like, configuration, to the fibers as will be hereinafter described. The fibers are further characterized by their wool-like fluffy appearance and texture when formed into a nonwoven structure such as, for example, a mat or batting. As will become apparent, the greater the amount of coil-like fibers present in the structure, the greater will be the wool-like texture and resilience. The fibers may be blended with noncarbonaceous fibers or linear carbonaceous fibers.

2

The carbonaceous fibers are preferably nonlinear but may also include blends of nonlinear and linear carbonaceous fibers. The carbonaceous fiber structures of the invention possess excellent antistatic, antisloshing and flame arresting properties. Advantageously, the structure comprises a multiplicity of resilient carbonaceous fibers having a sinusoidal or coil-like shape with a reversible deflection ratio of at least about 1.2:1, an aspect ratio (1/d) of greater than 10:1 and a LOI (limited oxygen index) of greater than 40. The carbonaceous fiber structure when in the form of a wool like fluff is extremely lightweight and has a bulk density of from 2.4 to 32 kg/m$^3$, preferably from 2.4 to 8.0 kg/m$^3$.

More particularly, the invention resides in a flame arresting structure for use with containers for holding flammable fluids comprising a multiplicity of nonflammable, substantially irreversibly heat set, carbonaceous fibers having an LOI value greater than 40, said carbonaceous fibers being present in an amount effective for arresting a flame front.

The carbonaceous fiber structure is present on the inside of the container in an amount effective to prevent sloshing of the flammable fluid and also to prevent the buildup of an electrostatic charge in the container which could generate a spark to ignite the fuel. The fuel in the container may be entirely liquid or entirely gaseous. When the fuel is in a liquid form, there is usually a zone above the liquid level in the container which is in a gaseous state and, therefore, is also highly flammable. Optionally, the carbonaceous fiber structure is also present on the outside of the container, i.e. on at least one outer suface of the container, in an amount effective to arrest a flame front extending from a rupture in the container. The term "amount effective" used herein is used in a broad sense since the amount depends on the density of the fibrous structure and its location on the inside or on the outside of the container. The density of the fibrous structure, in turn, will vary depending upon the amount of fluid flow desired. The fluid flow from a fuel tank to power an aircraft engine, for example, may be relatively high, requiring a relatively high flow of fuel from the tank.

The invention also resides in a container for holding a flammable liquid and/or gas, wherein, the inside of said container contains a carbonaceous fiber structure in an amount sufficient to substantially retard sloshing of the liquid within the container but without preventing the flow of liquid from the container, and wherein at least a portion of an outer surface of said container is covered with a flame arresting carbonaceous fiber structure in an amount sufficient to arrest a flame front of the flammable liquid and/or gas passing through a rupture in the container.

The present invention is specifically concerned with structures comprising a multiplicity of the nonflammable carbonaceous fibers which are particularly identified by their degree of carbonization and/or their degree of electrical conductivity in the determination of the particular use for which they are most suited.

Carbonaceous fibers that are derived from nitrogen containing polymeric materials, such as an acrylic based polymer, generally have a nitrogen content of from 5 to 35 percent, preferably from 16 to 25 percent, more preferably from 18 to 20 percent.

The "electrical resistance" of a carbonaceous fiber is determined by measurement on a 6K tow of fibers with the individual fibers having a nominal diameter of from 7 to 20 microns. The "specific resistivity is calculated by measurements as described in European Patent Application Serial No. 0199567.

The carbonaceous fibrous material which is utilized in the composite structures of this invention may be classified into three groups depending upon the particular use and the environment that the structures in which they are incorporated are placed.

In accordance with one embodiment of the invention, the carbonaceous fibers which are utilized in antistatic, flame arresting and antislosh applications are electrically nonconductive and possess no antistatic characteristics, i.e., they are not able to dissipate an electrostatic charge. the nonconductive fibers have an electrical resistance of greater than 4 x 10$^6$ ohms/cm and, correspondingly, a specific resistivity of greater than 10$^{-1}$ ohm-cm. When the nonconductive fibers are selected from an acrylic polymer, it was determined that the nitrogen content of such fibers was greater than about 18 percent.

In accordance with a second embodiment of the invention, the carbonaceous fibers which are utilized in the structures of the invention are partially electrically conductive, i.e., have a low degree of electrical conductivity, and a carbon content of less than 85 percent. The electrical resistance of these fibers is generally from 4 x 10$^6$ to 4 x 10$^3$ ohms/cm. Preferably, the carbonaceous fibers are derived from stabilized acrylic fibers and possess a nitrogen content of from 16 to 20 percent. The larger the amount of carbon content of the fibers utilized, the higher the degree of electrical conductivity. These higher carbon content filaments still retain a wool-like appearance when formed into a mat or a batting especially when the majority of the fibers are nonlinear in shape. Also, as will become apparent, the greater the percentage of nonlinear fibers in the structure, the greater is the resiliency or loft of the structure. As a result of the greater carbon content, the structures prepared with these fibers have greater static dissipating and antislosh properties and result in effective flame arresters even at higher temperatures of use.

3

In accordance with a third embodiment of the invention, the carbonaceous fibers which are utilized in the antislosh and/or flame arresting structures of the invention have a carbon content of at least 85 percent. Preferably, the filaments which are utilized are derived from stabilized acrylic fibers and have a nitrogen content of less than 16 percent. As a result of the still higher carbon content, structures prepared from such fibers have a higher electrical conductivity. That is, the resistance is less than $4 \times 10^3$ ohms/cm. Correspondingly, the specific resistivity is less than $10^{-1}$ ohms-cm.

The highly conductive fibers can be utilized in place of conventional straight or linear carbon fibers. Moreover, the coil-like carbonaceous fibers, when formed into a structure such as a mat or batting, provide a better ability to electrically ground and thus prevent the buildup of static electricity and the generation of sparks. A structure containing a greater amount of coil-like fibers, as opposed to sinusoidal or linear fibers, provides a more effective barrier against the spread of flame fronts.

Linear heat set carbonaceous fibers which are utilized in antistatic, antislosh and/or flame arresting applications are comprised of fibers formed from the same stabilized precursor material as the nonlinear fibers with the exception that the step of irreversibly heat setting (thermosetting) the fibers is done with the fiber precursor material held in a linear configuration. The linear carbonaceous fibers may be blended with and at least partially bonded to nonlinear thermoplastic or thermoset fibers or fiber blends in the form of a fluff, batting, web, or the like.

In accordance with the present invention, an open web or batting is provided consisting or randomly entangled, nonlinear carbonaceous fibers, as herein defined. The web is of a sufficient structural strength to maintain its open web shape in the presence of flammable liquids such as liquid hydrocarbons and thus prevent sloshing of the liquid as well as to be substantially noncompacting. Various degrees of structural ridigity and stiffness of the web can be obtained by bonding or curing a thermoplastic or thermoset material in the web. In addition, the product is fire or flame resistant and effectively acts as a wick and a flame block to prevent a mass of the flammable liquid entrapped in the web from rapidly spilling out, thus reducing the chance of explosion. The tightness of the web of the invention can be varied such as to provide an increased holdup or retardation of liquid flow, or of a looseness such as to permit a higher rate of liquid flow, depending on the particular use for the web. The web may also be fixed in its desired form and/or shape by cementing the juxtapositioned fibers one to the other leaving the web still very open, but of a more stable, less compressible body under load.

If desired, the structure may comprise a blend of linear carbonaceous fibers with thermoplastic or thermoset polymeric fibers or a fiber blend such as a polyester, or a blend of a high melting polyester with a smaller content of a lower melting polyester binder fiber in order to provide densification and/or some ridigity and integrity to the structure. It is also desirous for this structure to have a sufficient amount of carbonaceous fibers present to provide flame retardant properties.

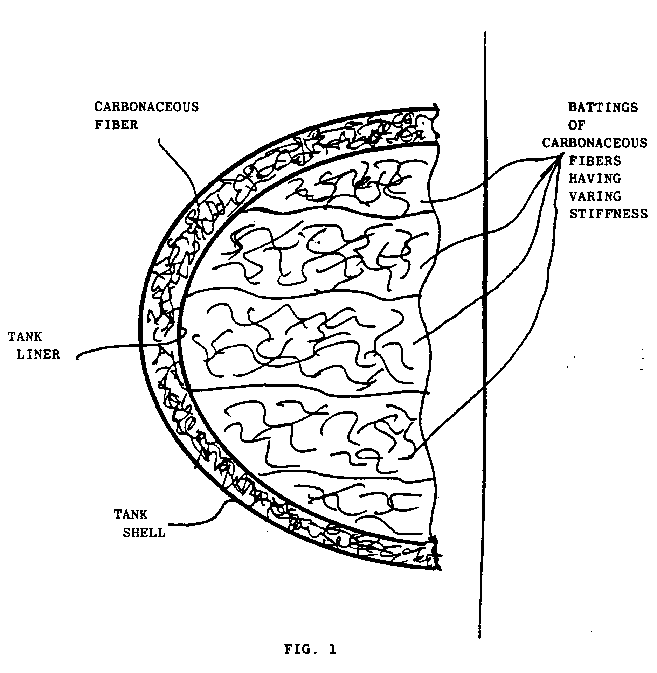

Figure 1 is a cross-sectional view of a container for flammable fluids illustrating one embodiment of the present invention.

The antislosh, flame arresting and/or antistatic fibrous structures of the invention are formed from nonflammable carbonaceous fibers, preferably fibers which are nonlinear and resilient, and which possess a reversible deflection ratio of greater than about 1.2:1 and an aspect ratio (1/d) of greater than 10:1. The nonlinear carbonaceous fibers may have a sinusoidal or a coil-like configuration or a more complicated structural combination of the two.

The fibers of the invention have a LOI value greater than 40, as determined by test method ASTM D 2863-77. The test method is also known as "oxygen index" or "limited oxygen index" (LOI).

The carbonaceous fibers of the invention are prepared by heat treating a carbonaceous precursor material such as that derived from acrylic materials, pitch based materials, such as petroleum or coal tar, or other carbonaceous polymeric materials which can be made into linear or nonlinear carbonaceous fibers or fibrous structures which are thermally stable and highly flame resistant.

For example, in the case of polyacrylonitrile (PAN) based fibers, the fibers are formed by melt or wet spinning a suitable liquid of a precursor material having a normal nominal diameter of from 4 to 25 micrometers. The precursor material is collected as an assembly of a multiplicity of continous filaments in tows and stabilized (by oxidation in the case of PAN based fibers) in the conventional manner. The oxidation stabilized tows (or staple yarn made from chopped or stretch broken fiber staple) may thereafter, in accordance with the present invention, be formed into a sinusoidal or other nonlinear shape by knitting or weaving the tow or yarn into a fabric or cloth (recognizing that other fabric forming and coil forming methods can be employed). The so-formed fabric or cloth is thereafter heat treated, with the fibers or fiber tow in a relaxed and unstressed condition, at a temperature of from 525°C to 750°C, in an inert atmosphere for a period of time sufficient to produce a heat induced thermoset reaction wherein additional cross-linking and/or a cross-chain cyclization reaction occurs between the original polymer chain. At the

4

lower temperature range of from 150°C to 525°C, the fibers are provided with a varying degree of temporary to permanent set while in the upper range of temperatures of from 525°C and above the fibers are provided with a substantially permanent or irreversible heat set. It is understood that other methods of shape formation such as crimping and coiling combined with thermosetting can be used.

What is meant by substantially permanently or irreversibly heat set is that the fibers possess a degree of irreversibility in their set nonlinear configuration. More particularly, once a carbonaceous fiber is heat set in a nonlinear shape, it can be stretched to a substantially linear shape but will always return to its nonlinear shape once the tension on the fiber is released. It is, of course, to be understood that the fiber or fiber assembly may be initially heat treated at the higher range of temperatures so long as the heat treatment is conducted while the coil-like and/or sinusoidal configuration is in a relaxed or unstressed state and under an inert, nonoxidizing atmosphere. As a result of the higher temperature treatment, a heat set coil-like or sinusoidal configuration or structure is imparted to the fibers, yarns, tows or threads. The resulting fibers, tows or yarns having the nonlinear structural configuration which are derived by deknitting the cloth, are subjected to other methods of treatment known in the art to create an opening. In such a procedure, the nonlinear fibers, yarn or tow of the cloth are separated into an entangled, wool-like fluffy material in which the individual fibers retain their coil-like or sinusoidal configuration yielding a fluff or batting-like body of considerable loft.

The fluff or batting of the invention may be utilized alone or may be provided with a suitable barrier layer of flexible sheet material or metal depending upon its desired used.

The stabilized fibers when permanently shaped into the desired structural configuration, e.g., by knitting, and thereafter heating at a temperature of greater than about 550°C retain their resilient and reversible deflection characteristics. It is to be understood that higher temperatures may be employed of up to about 1500°C, but the most flexible and the smallest loss of fiber breakage, when carded to produce the fluff, is found in those fibers and/or filaments that are heat treated to a temperature of from 525°C to 750°C.

When the precursor stabilized fiber is an acrylic fiber, the percentage nitrogen content is generally from 16 to 25 percent. The fibers are excellent for use where electrical antistatic properties are desired. The structures formed therefrom are lightweight, have low moisture absorbency, good abrasive strength together with good appearance and handle.

Carbonaceous fibers having a carbon content of at least 85 percent have superior thermal stability and flame arresting characteristics. The coil-like structure in the form of a fluff (when carded) provides a web which has good compressibility and resiliency while maintaining improved flame arresting and electrical grounding capability. The structure prepared with such fibers have particular utility in high flammability applications where spark or electrostatic generation cannot be tolerated and in areas of high temperatures.

The precursor stabilized acrylic filaments which are advantageously utilized in preparing the fibers of the structures are selected from the group consisting of acrylonitrile homopolymers, acrylonitrile copolymers and acrylonitrile terpolymers. The copolymers preferably contain at least about 85 mole percent of acrylonitrile units and up to 15 mole percent of one or more monovinyl units copolymerized with styrene, methylacrylate, methyl methacrylate, vinyl chloride, vinylidene chloride, vinyl pyridine and the like. Also, the acrylic filaments may comprise terpolymers wherein the acrylonitrile units are at least about 85 mole percent.

It is to be further understood that carbonaceous precursor starting materials may have imparted to them an electrically conductive property on the order of that of metallic conductors by heating the fiber fluff or the batting-like shaped material to a temperature above 1000°C in a nonoxidizing atmosphere. The electroconductive property may also be obtained from other selected starting materials such as pitch (petroleum or coal tar), polyacetylene, acrylonitrile based materials, e.g., a polyacrylonitrile copolymer (PANOX™ or GRAFIL-01™, trademarks of E. I. du Pont de Nemours & Co., Inc.), polyphenylene, polyvinylidene chloride resin (SARAN™, a trademark of The Dow Chemical Company) and the like.

A wool-life fluff of the carbonaceous fibers of the invention may be treated with an organic or inorganic binder, needle punched, bagged or adhered to a flexible or rigid support using any of the conventional materials and techniques depending upon the use and environment of the structure. The fluff may be placed on the inside of a structure, such as a fuel tank, or between structural parts, such as a container liner and outside skin, either in the form of a mat, web, felt, batting, or similar structure. Advantageously, various degrees of structural rigidity and stiffness of the carbonaceous structure can be obtained by incorporating a bonded or cured thermoplastic or thermoset polymeric material in the structure. This is preferably accomplished by blending thermoset or thermoplastic polymeric fibers with the carbonaceous fibers of the invention prior to formation of the structure followed by curing or thermobonding, or by coating the web or fluff with a thermoplastic resinous material followed by curing. Also, other stiffening fibers such as, for

5

example, high performance fibers may be blended with the carbonaceous fibers to enhance the stiffness and integrity of the web. Exemplary of high performance fibers are KEVLAR™ (an aromatic polyamide and a trademark of E. I. du Pontde Nemours & Co., Inc.), NOMEX™ (an aramid fiber and a trademark of E. I. du Pont de Nemours & Co., Inc.), meta aramid fibers, polybenzimidazole (PBI) fibers, and the like.

The coil-like or sinusoidal carbonaceous fibers and/or filaments are formed such that they are sufficiently electrically conductive to dissipate an electrostatic buildup before it achieves a dangerous level. In addition, the material is fire or flame resistant, effectively acts as a barrier to reduce the outflow of a flammable liquid from the container, and has the ability to act as a flame arrester should a leaking liquid from the container ignite, thus reducing the chance of explosion. The tightness of the web of the invention can be varied such as to provide a marked holdup or retardation of liquid flow, or of a looseness to permit a high rate of liquid flow. The web may also be fixed in its desired form and/or shape by cementing the juxtapositioned fibers one to the other leaving the web still very open but of a more stable, less compressible body under load.

In another preferred embodiment, it is desirous to have a layered construction, such as that illustrated in the figure. In this embodiment, layers of the structure of the invention having varying degrees of stiffness, and hence varying degrees of fuel flow rates, are placed within the container, and a nonstiffened structure is placed between the inner liner and outer wall of the container to retard the leakage of fuel and act as a flame arrester, should the container rupture.

If desired, the structure may also comprise a thermoplastic polymeric fiber, such as polyester fiber, or the like, in order to provide densification, shape retention and extra rigidity of the structure.

Exemplary of the present invention are the following examples.

Example 1

In this example, a fluff or wool-like structure was prepared which is particularly useful in antislosh and flame arresting applications where electrical grounding of the structure is required.

Following the procedure disclosed in European Patent Application Serial No. 0199567, published October 29, 1986, to F. P. McCullough, et al entitled, "Carbonaceous Fibers with Spring-Like Reversible Deflection and Method of Manufacture," a 3K OPF (i.e., oxidized polyacrylic fiber having 3000 filaments) PANOX™ stabilized tow was knit on a Singer flat bed knitting machine at a rate of 4 stitches/cm and was then heat treated at a temperature of 950°C. The cloth was deknitted and the tow (which has a coil elongation or reversible deflection ratio of greater than 2:1) was cut into 7.5 cm length staple. The cut staple was then carded on a Platt Miniature carding machine to produce a wool-like fluff having fibers ranging from 2.5 cm to 6.5 cm in length. The wool-like fluff had a high electrical conductivity (a resistance of less than 40 ohms/cm) over any length of up to 60 cm tested.

In lieu of PANOX™, there may be employed stabilized pitch based fibers or a copolymer or terpolymer of polyacrylonitrile.

Example 2

In this example, a fluff or wool-like structure was prepared which is particularly useful in antislosh and flame arresting applications where electrical grounding of the structure is required.

In a similar manner to Example 1, a portion from the same knit sock was heat treated at a temperature of 1550°C. The cloth itself and the deknitted tow had a very high electrical conductivity. On carding 15 cm lengths of cut tow, a wool-like fluff was obtained which had fiber lengths of from 2.5 to 7.5 cm with average lengths of 5 cm. Thus, carding of a deknitted fabric of a continuous filament tow which has been subjected to a temperature of above 1000°C is still capable of producing a wool-like fluff product.

Example 3

In this example, a structure effective as an antislosh, antistatic and flame arrester capable of arresting flame fronts from gasoline or naphtha vapors was produced as follows:

Using the procedure of example 1, a 6K OPF tow was knitted into a fabric, the fabric treated at 650°C until it was thermally set and thereafter deknitted to produce a nonlinear carbonaceous fiber tow which was then cut into staple of from 14 to 17 cm nominal lengths. This so-cut staple was opened on a Shirley opener and further processed on a Rando Webber machine, an air laying system for producing nonwoven batting. The feed plate and combing roll were spaced at a distance of 0.3 mm and dispersed into the chamber using a 1200 rpm setting on the fan. Approximately 50 percent of 6 dernier polyester fiber was blended with opened, nonlinear carbonaceous fibers in the preblending section of the Rando Webber. The resulting batting was passed through a Benz hot air oven, held at a temperature of 260°C, at a rate of 2 m/minute resulting in an oven time of about 1 minute. This was sufficient to soften the polyester fibers to achieve a light bonding of the carbonaceous fibers in the web. This structure had excellent antistatic and stiffness properties, and was found to be especially useful in applications where good fuel flow rates are required.

Example 4

A. In a manner similar to the procedure described in Example 3, the cut fibers were treated in a Shirley opener and then a Rando Webber air laying system, but with approximately 1 percent low melting EAA (ethylene acrylic acid) binder fibers added.

B. The fluff and/or batting of Part A was partially coated with an epoxy resin to form structures of varying rigidity. The fluff or batting was placed on an expanded metal screen in a laboratory hood. A series of samples designated as Samples A to E were sprayed on all sides with a novolac epoxy resin (D.E.N.™ 438, a trademark of The Dow Chemical Company) as a 20 percent solution in acetone to which had been added (in a ratio of 28 to 100 of the amount of the epoxy used) methylene dianiline as a hardening agent. Sample F was sprayed on all sides with an aliphatic diepoxide resin (D.E.R.™ 732) as a 20 percent solution in methyl ethyl ketone to which had been added (in a ratio of 31:100 of the amount of the epoxide used) methylene dianiline as a hardening agent. After driving the acetone out of each sample under a heat lamp, the coated sample was placed in an oven at 105°C. The temperature was increased to 125°C where it remained for approximately 1 hour. The oven temperature was then raised to 170°C and held thereat for an additional hour. Upon cooling, each sample was weighted to determine the percent resin content. Electrical resistance measurements were also made on each sample. The results are summarized in Table I as follows:

## TABLE I

| Sample | Epoxy:Fiber Ratio | Surface R (megaohm/sq.) |
|--------|-------------------|-------------------------|
| A | 3.12:1 | 300 |
| B | 1.26:1 | 300 |
| C | 1.31:1 | 200 |
| D | 1.53:1 | 200 |
| E | 0.35:1 | 200 |
| F | 1.50:1 | 200 |

The stiffness and rate of fuel flow through the battings followed the relative amount of epoxy coating on each batting. Batting A had the greatest stiffness and had the best fuel flow rate whereas Batting E was the softest and had the least stiffness and thus had a higher fuel flow rate. This example also surprisingly shows that the battings retain good antistatic properties over a large coating range of epoxy coating.

Example 5

The batting of Example 4 was processed on a Hunter Fiber Locker to obtain a mechanical bonding by the needle punching process. The resulting structure was suitable as an antistatic, antislosh and flame arresting outer container liner structure in a container for flammable fluids.

Example 6

Following the procedure disclosed in European Application Serial No. 0199567, a 6K OPF (i.e., 6000 filament oxidized polyacrylic fiber) PANOX™ stabilized tow was knit into a plain jersey fabric on a flat bed knitting machine at a rate of 3 stitches/cm and was then heat treated at a temperature of 550°C. The cloth was deknitted and the tow (which had a coil elongation or reversible deflection ratio of greater than 1.2:1) was cut into 7.5 cm length staple. The cut staple was then carded on a Platt Miniature carding machine to produce a wool-like fluff having fibers ranging from 6.5 cm to 7.5 in length. The wool-like fluff had a measurable electrical conductivity (a resistance of less than 125K ohms/2.5 cm). A small portion of the resulting fluff (0.14 g) was placed in a 75 ml laboratory widemouthed bottle. No compression was used to place more than the free volume of the fluff into the bottle. The bottle was then approximately 90 percent filled with 55 g of a highly refined liquid hydrocarbon (kerosene) and sealed. The electrical resistance across 2.5 cm of kerosene was greater than 20 megaohms with no fluff present and less than 125K ohms with fluff present (this value of less than 125K ohms across 2.5 cm is well within the antistatic range). The bottle was shaken and inverted several times and trapping of air bubbles and a significant antisloshing effect due to the fluff was observed. During the inversion, the carbonaceous fiber batting was compressed by about 10 percent. The bottle was opened and the kerosene was poured freely from the bottle. This example showed that the fluff structure is free supporting in a container of short head height (about 7.5 cm) and is effective as an antislosh and antistatic structure.

Example 7

This example was run to show what effect increasing the density of a carbonaceous fiber structure which was lightly coated with an epoxy resin would have on the amount of liquid retained by the structure.

A 0.48 g portion of Sample E from Example 4B (epoxy resin to fiber ratio of 0.35:1) was loaded into a 125 ml bottle and the bottle filled with 100.12 g of kerosene. The bottle was turned upside down and the kerosene was allowed to flow out of the bottle for a period of about 30 seconds. The kerosene which flowed from the bottle weighed 91.65 g. Thus, 8.47 grams were retained in the bottle. A second bottle was filled with 0.99 g of the coated sample and the bottle filled with 96.6 grams of kerosene. The bottle was turned upside down and the kerosene was allowed to run out of the bottle for 30 seconds. 79.71 grams of the kerosene flowed from the bottle. Thus, 16.89 grams or 17.3 percent by weight was retained by the structure in the bottle. This example showed that when the structure is lightly coated with an epoxy resin, an increase in the density of the structure increases the amount of liquid retained by the structure.

Examples 8 and 9

These examples were run to show the effect of stiffness caused by coating the carbonaceous fiber structure versus the fuel flow characteristics.

In a first experiment, 4.63 g of an uncoated carbonaceous fiber batting of Example 4A was placed in a 125 ml widemouthed laboratory bottle to which was added 85 g of kerosene. The bottle containing the uncoated structure of the invention was rotated and shaken several times. The bottle was then opened and inverted for 30 seconds, during which time interval 1.7 g, or 2 percent, of the kerosene flowed out of the bottle. Approximately 98 percent of the kerosene was retained by the structure.

In a second experiment, 4.63 g of a coated carbonaceous fiber batting of Sample F of Example 4B was placed in the 125 ml widemouthed laboratory bottle to which was added 85.57 g of kerosene. The bottle containing the coated structure of the invention was rotated and shaken several times. The bottle was then opened and inverted for 30 seconds during which time interval 82.46 g, or 95.3 percent, of the kerosene flowed out of the bottle. Less than 5 percent of the kerosene was retained by the stiffened coated structure.

These experiments show that the fuel retention is a function of the stiffness of the structure and not the mass of the structure per se. The uncoated batting would be therefore very useful as an outer liner for a fuel tank to retard spillage of fuel in the case of tank penetration or rupture whereas the coated example would be very useful inside of a fuel tank where anitslosh, flame arresting and antistatic properties and good flow rates are required.

Example 10-13

In another series of examples, the flow rates and antisloshing characteristics of various embodiments of the invention were demonstrated and compared to a currently used foam structure. A nominal 15 cm OD PYREX™ (a trademark of Corning Glass Works) glass tube 1.8 m long was sealed at both ends with removable plates having valves fitted in the plates. The tube was suspended at its central portion in a trunnion assembly so that the entire tube could be rocked and/or rotated 360 degrees. This tube was packed with each example of material to be tested for slosh and drain characteristics.

The materials tested in this series of examples were 600 g of standard blue polyether urethane reticulated foam, a 400 g sample of example 4A, and Samples A (275 g), B (356 g), and D (300 g) of Example 4B, the latter four examples containing embodiments of the material of the invention. The sample of Example 4A had no stiffener coating whereas the remaining samples had different amounts of coatings as described in Table I. The tube was filled with 31.5 liters of kerosene and partial drain rates measured. The tube was then rotated 180 degrees to measure the relative anitslosh characteristics for each example. "Time 1" in Table II represents the time in seconds for 80 percent of 10.5 liters of kerosene to move from the top to the bottom of the 1.8 m tube whereas "Time 2" represents the time in seconds for 90 percent of the 10.5 liters (33 percent full tank) of kerosene to slosh back from top to bottom after rotation. On a total 31.5 liter drain test Example 13 retained 0.35 liters of fluid or only 1.1 percent fuel retention.

## TABLE II

| | | | | Slosh Test (sec) | |
|---|---|---|---|---|---|
| Example No. | Sample | Fraction Drained | Time (sec) | Time 1 | Time 2 |
| Comparative Sample | Blue Foam | 0.33 0.67 | 60 - | - 60 | - 80 |
| 10 | 4A No coating | 0.33 0.67 | >2000 | >1800 | |
| 11 | A 3.12:1 | 0.33 0.67 | 26 71 | 48 | 74 |
| 12 | B 1.26:1 | 0.33 0.67 | 35 105 | 102 | 115 |
| 13 | C 1.53:1 | 0.33 0.66 1.00 | 32 88 160 | - | - |

Example 14

A 6K linear tow of PANOX™ oxidized polyacrylic fiber (OPF) was heat treated at 650°C similar to the heat treatment described in Example 3. This heat set linear fiber tow was cut into 7.5 cm staple length, opened with turbulent air and blended with 75 percent of 6 dernier polyester fiber and made into a 2.5 cm thick batting having a bulk density of approximately 9 kg/m$^3$. This batting was then thermally bonded in a Benz hot air oven in a manner similar to that of Example 3. This material passed the vertical burn test as described in ASTM test procedure 14 CFR 25.853b and had an electrical resistance of less than 1 megaohm/2.5 cm (in the effective antistatic range).

This material was shown to be effective as a flame retardant material useful as an antislosh, antistatic flame arresting structure.

Example 15

A 6K linear fiber tow of PANOX™ oxidized polyacrylic fiber (OPF) was heat treated at 650°C similar to the heat treatment described in Example 3. This heat set linear tow was cut into 7.5 cm staple length, opened with turbulent air and blended with 60 percent of 6 denier high melting polyester fiber, 15 percent low melting polyester binder fiber, and made into a 2.5 cm thick batting having a density of approximately 9 kg/m$^3$. This batting was then thermally bonded in a Benz hot air oven in a manner similar to that of Example 3, with the exception that the temperature of the oven was set in between the melting points of the regular polyester fiber and the lower melting polyester binder fiber. This allowed only the binder fiber to melt and created a batting with a lower density and higher loft than that made in Example 14. This material passed the vertical burn test as described in 14 CFR 25.853b and had an electrical resistance of less than 1 megaohm per 2.5 cm (in the effective antistatic range). This material was shown to be effective as a flame retardant material useful as an antislosh, antistatic, flame arresting structure.

**Claims**

1.   Use of nonflammable, substantially irreversibly heat set, carbonaceous fibers having a LOI value greater than 40 in a flame arresting structure for use with containers for holding flammable fluids.

2.   The use of the fibers of claim 1, wherein said carbonaceous fibers are selected from nonlinear fibers or blends of nonlinear and linear fibers in which the linear fibers are present in the blend in an amount of less than 15 percent, based on the total weight of the fibers.

3.   The use of the fibers of claim 2, wherein said nonlinear carbonaceous fibers have a sinusoidal or coil-like configuration and a reversible deflection ratio of at least 1.2:1.

4.   The use of the fibers of claim 1, 2 or 3, wherein said carbonaceous fibers have an aspect ratio of greater than 10:1 and a nitrogen content of from 5 to 35 percent.

5.   The use of the fibers of any one of the preceding claims, wherein said carbonaceous fibers are electrically conductive, have a carbon content of greater than 85 percent by weight, an electrical resistance of less than $4 \times 10^3$ ohms/cm, and a specific resistivity of less than $10^{-1}$ ohms-cm.

6.   The use of the fibers of any one of claims 1 to 4, wherein said carbonaceous fibers are electrically nonconductive and do not possess any electrostatic dissipating characteristics, said fibers having a carbon content of greater than 65 percent but less than 85 percent by weight, an electrical resistance of greater than $4 \times 10^6$ ohms/cm, and a specific resistivity of greater than $10^{-1}$ ohms-cm.

7.   The use of the fibers of any one of claims 1 to 4, wherein said carbonaceous fibers have a low electrical conductivity and electrostatic dissipating characteristics, said fibers having an electrical resistance of from $4 \times 10^6$ to $4 \times 10^3$ ohms/cm.

8.   The use of the fibers of any one of the preceding claims, wherein said carbonaceous fiber structure is in the form of a wool-like fluff, webbing, matting or batting comprising a multiplicity of entangled continuous or staple carbonaceous fibers having a bulk density of from 2.4 to 32.0 kg/m$^3$.

9.   The use of the fibers of any one of the preceding claims, wherein said carbonaceous fiber structure contains an organic or inorganic binder.

10.   The use of the fibers of any one of claims 1 to 8, wherein said carbonaceous fibers structure is rigidified by bonding the carbonaceous fibers in the structure one to another with a thermoset or thermoplastic polymeric binder to produce a structure having structural integrity and to maintain an open porous structure in the presence of said flammable fluid.

11.   The use of the fibers of claim 9 or 10, wherein said carbonaceous fibers contain less than 25 percent by weight of said binder, and said binder is selected from polyester fibers or an epoxy resin.

12.   The use of the fibers of any one of the preceding claims, wherein said carbonaceous fibers are blended with high performance polymeric fibers.

13.   The use of the fibers of any one of the preceding claims, wherein said carbonaceous fibers are derived from oxygen stabilized acrylic fibers having a diameter of from 4 to 25 microns.

14.   The use of the fibers of claim 13, wherein said acrylic fibers are selected from acrylonitrile homopolymers, acrylonitrile copolymers and acrylonitrile terpolymers, wherein said copolymers and terpolymers contain at least 85 mole percent acrylic units and up to 15 mole percent of one or more monovinyl units copolymerized with another polymer.

15. A container for holding a flammable liquid and/or gas, wherein the inside of said container contains a carbonaceous fiber structure, said carbonaceous fiber structure comprising a multiplicity of substantially irreversibly heat set, nonflammable, continuous or staple carbonaceous fibers having a LOI value of greater than 40, wherein said carbonaceous fiber structure is in the form of a wool-like fluff, webbing, matting or batting, and wherein optionally at least a portion of an outer surface of said container is covered with the same carbonaceous fiber structure

16. The container of claim 15, wherein the carbonaceous fiber structure within the container has a bulk density of from 2.4 to 32 kg/m$^3$ and is provided with a thermoset or thermoplastic polymeric binder to form a web having structural integrity to maintain an open porous structure in the presence of a liquid.

17. The container of claim 15 or 16, wherein said carbonaceous fibers have a coil-like and/or sinusoidal shape with a reversible deflection ratio of greater than 1.2:1.

18. A process for providing a flame arresting container for a flammable fuel characterized in that said container is provided with a fibrous structure comprising a multiplicity of nonflammable, substantially irreversibly heat set, carbonaceous fibers having a LOI value greater than 40.

19. The process of claim 18, including the step of positioning said fibrous structure within the container to prevent sloshing of the fuel and to dissipate the buildup of an electrostatic charge in the container.

20. The process of claims 18 or 19, including the step of covering at least a portion of an outer surface of said container with said carbonaceous fiber structure in an amount sufficient to arrest a flame front of the flammable liquid and/or gas from passing through a rupture in the container.

## Patentansprüche

1. Verwendung von nicht-entflammbaren, im wesentlichen irreversibel heißfixierten, kohlenstoffhaltigen Fasern mit einem LOI-Wert von mehr als 40 in einer feuerhemmenden Struktur zur Anwendung bei Behältern für die Aufnahme entflammbarer Fluids.

2. Verwendung der Fasern nach Anspruch 1, worin die kohlenstoffhaltigen Fasern aus nicht-linearen Fasern oder Mischungen von nicht-linearen Fasern und linearen Fasern ausgewählt sind, worin die linearen Fasern in der Mischung in einer Menge von weniger als 15 % auf Basis des Fasergesamtgewichts vorhanden sind.

3. Verwendung der Fasern nach Anspruch 2, worin die nicht-linearen kohlenstoffhaltigen Fasern eine sinusförmige oder spiralförmige Konfiguration und ein reversibles Auslenkungsverhältnis von mindestens 1,2 : 1 besitzen.

4. Verwendung der Fasern nach Anspruch 1, 2 oder 3, worin die kohlenstoffhaltigen Fasern ein Aspektverhältnis von größer als 10 : 1 und einen Stickstoffgehalt von 5 bis 35 % besitzen.

5. Verwendung der Fasern nach einem der vorhergehenden Ansprüche, worin die kohlenstoffhaltigen Fasern elektrisch leitfähig sind, einen Kohlenstoffgehalt von mehr als 85 Gew.-%, einen elektrischen Widerstand von weniger als 4 x 10$^3$ Ohm/cm und einen spezifischen Widerstand von weniger als 10$^{-1}$ Ohm-cm besitzen.

6. Verwendung der Fasern nach einem der Ansprüche 1 bis 4, worin die kohlenstoffhaltigen Fasern nicht elektrisch leitfähig sind und keine elektrostatische Ableitungseigenschaften besitzen, wobei die Fasern einen Kohlenstoffgehalt von mehr als 65 Gew.-%, aber weniger als 85 Gew.-%, einen elektrischen Widerstand von mehr als 4 x 10$^6$ Ohm/cm und einen spezifischen Widerstand von mehr als 10$^{-1}$ Ohm-cm besitzen.

7. Verwendung der Fasern nach einem der Ansprüche 1 bis 4, worin die kohlenstoffhaltigen Fasern eine geringe elektrische Leitfähigkeit und geringe elektrostatische Ableitungseigenschaften besitzen, wobei die Fasern einen elektrischen Widerstand von $4 \times 10^6$ bis $4 \times 10^3$ Ohm/cm besitzen.

8. Verwendung der Fasern nach einem der vorhergehenden Ansprüche, worin die kohlenstoffhaltige Faserstruktur in Form eines wolleartigen Flaums, eines Gewebes, einer Matte oder einer Watte ist und eine Vielzahl von verfitzten, kohlenstoffhaltigen kontinuierlichen Fasern oder Stapelfasern mit einer Massendichte von 2,4 bis 32,0 kg/m$^3$ umfaßt.

9. Verwendung der Fasern nach einem der vorhergehenden Ansprüche, worin die kohlenstoffhaltige Faserstruktur einen organischen oder anorganischen Binder enthält.

10. Verwendung der Fasern nach einem der Ansprüche 1 bis 8, worin die kohlenstoffhaltige Faserstruktur durch Bindung der kohlenstoffhaltigen Fasern in der Struktur aneinander mit einem thermofixierten oder thermoplastischen Polymerbinder versteift wird, um eine Struktur mit struktureller Beständigkeit zu erzeugen und um in Gegenwart des entflammbaren Fluids eine offenporige Struktur beizubehalten.

11. Verwendung der Fasern nach Anspruch 9 oder 10, worin die kohlenstoffhaltigen Fasern weniger als 25 Gew.-% des Binders enthalten und der Binder aus Polyesterfasern oder einem Epoxidharz ausgewählt ist.

12. Verwendung der Fasern nach einem der vorhergehenden Ansprüche, worin die kohlenstoffhaltigen Fasern mit Hochleistungs-Polymerfasern vermischt werden.

13. Verwendung der Fasern nach einem der vorhergehenden Ansprüche, worin die kohlenstoffhaltigen Fasern von Sauerstoff-stabilisierten Acrylfasern mit einem Durchmesser von 4 bis 25 Mikron stammen.

14. Verwendung der Fasern nach Anspruch 13, worin die Acrylfasern aus Acrylnitril-Homopolymeren, Acrylnitril-Copolymeren und Acrylnitril-Terpolymeren ausgewählt sind, worin die Copolymere und Terpolymere mindestens 85 Mol-% Acryleinheiten und bis zu 15 Mol-% von einer oder mehreren mit einem anderen Polymer copolymerisierten Monovinyleinheiten enthalten.

15. Behälter zum Aufnehmen einer entflammbaren Flüssigkeit und/oder eines entflammbaren Gases, worin die Innenseite des Behälters eine kohlenstoffhaltige Faserstruktur enthält, wobei die kohlenstoffhaltige Faserstruktur eine Vielzahl von im wesentlichen irreversibel heißfixierten, nicht-entflammbaren, kohlenstoffhaltigen kontinuierlichen Fasern oder Stapelfasern mit einem LOI-Wert von mehr als 40 enthält, worin die kohlenstoffhaltige Faserstruktur in Form eines wolleartigen Flaums, eines Gewebes, einer Matte oder Watte ist, und worin gegebenenfalls mindestens ein Teil einer Außenoberfläche des Behälters mit der gleichen kohlenstoffhaltigen Faserstruktur bedeckt ist.

16. Behälter nach Anspruch 15, worin die kohlenstoffhaltige Faserstruktur innerhalb des Behälters eine Massendichte von 2,4 bis 32 kg/m$^3$ besitzt und mit einem thermofixierten oder thermoplastischen Polymerbinder versehen ist, um ein Gewebe mit einer strukturellen Beständigkeit zu bilden, so daß in Gegenwart einer Flüssigkeit eine offenporige Struktur beibehalten wird.

17. Behälter nach Anspruch 15 oder 16, worin die kohlenstoffhaltigen Fasern eine spiralförmige und/oder sinusförmige Gestalt mit einem reversiblen Auslenkungsverhältnis von mehr als 1,2 : 1 besitzen.

18. Verfahren zur Bereitstellung eines feuerhemmenden Behälters für einen entflammbaren Brennstoff, **dadurch gekennzeichnet,** daß der Behälter mit einer Faserstruktur versehen wird, die eine Vielzahl von nicht-entflammbaren, im wesentlichen irreversibel heißfixierten, kohlenstoffhaltigen Fasern mit einem LOI-Wert von mehr als 40 umfaßt.

19. Verfahren nach Anspruch 18, umfassend den Schritt des Positionierens der Faserstruktur innerhalb des Behälters, um ein Schwappen des Brennstoffs zu vermeiden und um den Aufbau einer elektrostatischen Ladung im Behälter abzuleiten.

20. Verfahren nach Anspruch 18 oder 19, umfassend den Schritt des Bedeckens von mindestens einem Teil einer Außenoberfläche des Behälters mit der kohlenstoffhaltigen Faserstruktur in einer ausreichenden Menge, um den Durchtritt einer Flammenfront der entflammbaren Flüssigkeit und/oder des entflammbaren Gases durch einen Riß im Behälter zu hemmen.

**Revendications**

1. Utilisation de fibres carbonées, non inflammables, sensiblement irréversiblement thermostabilisées, ayant une valeur de l'indice IOL supérieur à 40, dans une structure arrêtant la flamme destinée a être utilisée dans des récipients contenant des fluides inflammables.

2. Utilisation de fibres selon la revendication 1, dans laquelle lesdites fibres carbonées sont choisies parmi des fibres non rectilignes ou des mélanges de fibres rectilignes et non rectilignes dans lesquels les fibres rectilignes constituent au moins 15 % du mélange par rapport au poids total des fibres.

3. Utilisation de fibres selon la revendication 2, dans laquelle lesdites fibres carbonées non rectilignes ont une configuration sinusoïdale ou hélicoïdale et ont un rapport de déformation réversible au moins égal à 1,2:1.

4. Utilisation de fibres selon la revendication 1, 2 ou 3, dans laquelle lesdites fibres carbonées ont un rapport d'allongement supérieur à 10:1 et une teneur en azote comprise entre 5 et 35 %.

5. Utilisation de fibres selon l'une quelconque des précédentes revendications, dans laquelle lesdites fibres carbonées sont électroconductrices, ont une teneur en carbone supérieure a 85 % en poids, une résistance électrique inférieure à $4 \times 10^3$ Ω/cm, et une résistivité spécifique inférieure à $10^{-1}$ Ω-cm.

6. Utilisation de fibres selon l'une quelconque des revendications 1 à 4, dans laquelle lesdites fibres carbonées sont non électroconductrices et ne possèdent aucune propriété de dissipation électrostatique, lesdites fibres ayant une teneur en carbone supérieure a 65 % mais inférieure à 85 % en poids, une résistance électrique supérieure à $4 \times 10^6$ Ω/cm et une résistivité spécifique supérieure à $10^{-1}$ Ω-cm.

7. Utilisation de fibres selon l'une quelconque des revendications 1 à 4, dans laquelle lesdites fibres carbonées ont des caractéristiques de dissipation électrostatique et de conductivité électrique faibles, lesdites fibres ayant une résistance électrique comprise entre $4 \times 10^6$ et $4 \times 10^3$ Ω/cm.

8. Utilisation de fibres selon l'une quelconque des précédentes revendications, dans laquelle ladite structure en fibres carbonées à la forme d'un fluff semblable à de la laine, d'une toile à sangles, d'un mat ou d'une ouate en feuille contenant une multiplicité de fibres carbonées emmêlées, continues ou en brins, ayant une densité en vrac comprise entre 2,4 et 32,0 kg/m$^3$.

9. Utilisation de fibres selon l'une quelconque des précédentes revendications, dans laquelle ladite structure en fibres carbonées contient un liant organique ou inorganique.

10. Utilisation de fibres selon l'une quelconque des revendications 1 à 8, dans laquelle ladite structure en fibres carbonées est rigidifiée en liant les fibres carbonées dans la structure les unes aux autres avec un liant polymère thermoplastique ou thermodurci pour produire une structure présentant une intégrité structurale et pour maintenir une structure poreuse ouyerte en présence dudit fluide inflammable.

11. Utilisation de fibres selon la revendication 9 ou 10, dans laquelle lesdites fibres carbonées contiennent moins de 25 % en poids dudit liant, et ledit liant est choisi parmi des fibres polyester ou une résine époxy.

12. Utilisation de fibres selon l'une quelconque des précédentes revendications, dans laquelle lesdites fibres carbonées sont mélangées à des fibres polymères hautement performantes.

13. Utilisation de fibres selon l'une quelconque des précédentes revendications, dans laquelle lesdites fibres carbonées dérivent de fibres acryliques stabilisées à l'oxygène ayant un diamètre compris entre 4 et 25 μm.

**14.** Utilisation de fibres selon la revendication 13, dans laquelle lesdites fibres acryliques sont choisies parmi les homopolymères d'acrylonitrile, les copolymères d'acrylonitrile et les terpolymères d'acrylonitrile, dans lesquels lesdits copolymères et terpolymères contiennent au moins 85 % en mole de motifs acryliques et jusqu'à 15 % en mole d'un ou plusieurs motifs monovinyles copolymérisés avec un autre polymère.

**15.** Récipient pour contenir un liquide et/ou un gaz inflammable, dans lequel l'intérieur dudit récipient contient une structure en fibres carbonées, ladite structure en fibres carbonées comportant une multiplicité de fibres carbonées, sensiblement irréversiblement thermostabilisées, non-inflammables, continues ou en brin, ayant une valeur de l'indice IOL supérieure à 40, dans laquelle ladite structure en fibres carbonées a la forme d'un fluff semblable à de la laine, d'une toile à sangles, d'un mat ou d'une ouate en feuille, et dans laquelle, éventuellement, au moins une partie de la surface extérieure dudit récipient est recouverte par la même structure en fibres carbonées.

**16.** Récipient selon la revendication 15, dans lequel la structure en fibres carbonées à l'intérieur du récipient a une densité en vrac comprise entre 2,4 et 32 kg/m$^3$ et comporte un liant polymère thermodurci ou thermoplastique pour former une sangle ayant une intégrité structurale afin de maintenir une structure poreuse ouverte en présence d'un liquide.

**17.** Récipient selon la revendication 15 ou 16, dans lequel lesdites fibres carbonées ont une forme hélicoïdale et/ou sinusoïdale avec un rapport de déformation réversible supérieur à 1,2:1.

**18.** Procédé de fabrication d'un récipient arrêtant la flamme destiné à un combustible inflammable, caractérisé en ce que ledit récipient est muni d'une structure fibreuse contenant une multiplicité de fibres carbonées non inflammables, sensiblement irréversiblement thermostabilisées, ayant une valeur de l'indice IOL supérieur à 40.

**19.** Procédé de la revendication 18, incluant l'étape qui consiste à placer ladite structure fibreuse à l'intérieur du récipient pour empêcher le ballottement du combustible et pour dissiper la charge électrostatique qui s'accumule dans le récipient.

**20.** Procédé selon la revendication 18 ou 19, incluant l'étape qui consiste à recouvrir au moins une partie de la surface extérieure dudit récipient avec ladite structure en fibres carbonées en une quantité suffisante pour empêcher qu'un front enflammé de liquide et/ou de gaz inflammable traverse un crevassement dans le récipient.

CARBONACEOUS
FIBER

BATTINGS
OF
CARBONACEOUS
FIBERS
HAVING
VARING
STIFFNESS

TANK
LINER

TANK
SHELL

FIG. 1